# EUROPEAN PATENT APPLICATION

(11) **EP 3 275 360 A1**
(43) Date of publication of application: **31.01.2018**
(21) Application number: 16875177.4
(22) Date of filing: 08.09.2016
(51) Int. Cl.: A61B 1/00, A61B 1/04, G02B 23/24, G02B 23/26

(54) **STEREOSCOPIC IMAGING UNIT**

(30) Priority: 17.12.2015 JP 2015246015
(71) Applicant: Olympus Corporation, Hachioji-shi, Tokyo 192-8507 (JP)
(72) Inventor: UNSAI Hiroshi, Hachioji-shi Tokyo 192-8507 (JP); ICHIHARA Hirokazu, Hachioji-shi Tokyo 192-8507 (JP); FUJII Toshiyuki, Hachioji-shi Tokyo 192-8507 (JP); NISHIHARA Teruyuki, Hachioji-shi Tokyo 192-8507 (JP); IMAI Mayumi, Hachioji-shi Tokyo 192-8507 (JP); ARAI Jumpei, Hachioji-shi Tokyo 192-8507 (JP); SATO Masahiro, Hachioji-shi Tokyo 192-8507 (JP)
(74) Representative: Gunzelmann, Rainer
(86) International application number: PCT/JP2016/076432
(87) International publication number: WO 2017/104191

(57) **Abstract**

A stereo image pickup unit includes a first image pickup device 321 configured to receive a first optical image formed by a first objective optical system 311, a second image pickup device 32r configured to receive a second optical image formed by a second objective optical system 31r, a single centering glass 34 that is disposed on optical paths of the respective first and second optical images and to which light receiving surfaces 321a and 32ra of the respective first and second image pickup devices 321 and 32r are positioned and fixed through bonding, and a holding frame 35 that holds the first and second image pickup devices 321 and 32r through the centering glass 34.

## Description

### Technical Field

The present invention relates to a stereo image pickup unit that acquires two picked-up images having parallax.

### Background Art

In recent years, in a field of medical endoscopes and industrial endoscopes, a need for stereoscopic observation of a subject with use of a stereo image pickup unit has been increasing.

As a configuration of a distal end portion of an endoscope using the stereo image pickup unit, for example, Japanese Patent Application Laid-Open Publication No. H8-29701 discloses a technology in which an objective lens unit is fitted into a transparent hole provided on a distal end member and a CCD unit is further fitted into the transparent hole. Two objective lens systems (objective optical systems) are integrally mounted on the objective lens unit, and two CCDs (image pickup devices) are integrally mounted on the CCD unit. For example, as disclosed in Japanese Patent Application Laid-Open Publication No. H8-29701, each of the image pickup devices is typically positioned and fixed to a holder through an individual centering glass. In other words, the centering glass corresponding to each of the image pickup devices is held by the holder, and each of the image pickup devices is positioned on the corresponding centering glass, and is then bonded to the centering glass, thereby being held.

Incidentally, the stereo image pickup unit of the type tends to have a complicated configuration as compared with a monocular image pickup unit that includes a single objective optical system and a single image pickup device.

In addition, in the stereo image pickup unit of the type, it is necessary to optimize parallax between two picked-up images. In particular, in an endoscope that enlarges a subject from a short distance and picks up an image of the subject, a distance between optical axes tends to become wide and to excessively enhance three-dimensional appearance.

The present invention is made in consideration of the above-described circumstances, and an object of the present invention is to provide a stereo image pickup unit that acquires two picked-up images with appropriate parallax through a simple configuration.

### Disclosure of Invention

### Means for Solving the Problem

A stereo image pickup unit according to an aspect of the present invention includes: a first image pickup device configured to receive a first optical image formed by a first objective optical system; a second image pickup device configured to receive a second optical image formed by a second objective optical system that is paired with the first objective optical system; a single optical member that is disposed on optical paths of the respective first and second optical images and to which light receiving surfaces of the respective first and second image pickup devices are positioned and fixed through bonding; and a holding frame that holds the first and second image pickup devices through the optical member.

### Brief Description of the Drawings

Fig. 1 is a perspective view illustrating an entire configuration of an endoscope system;
Fig. 2 is an end surface view of a distal end portion of an endoscope;
Fig. 3 is a cross-sectional diagram taken along line III-III in Fig. 2;
Fig. 4 is an enlarged cross-sectional diagram of a stereo image pickup unit; and
Fig. 5 is an exposed perspective view illustrating the stereo image pickup unit from proximal end side.

### Best Mode for Carrying Out the Invention

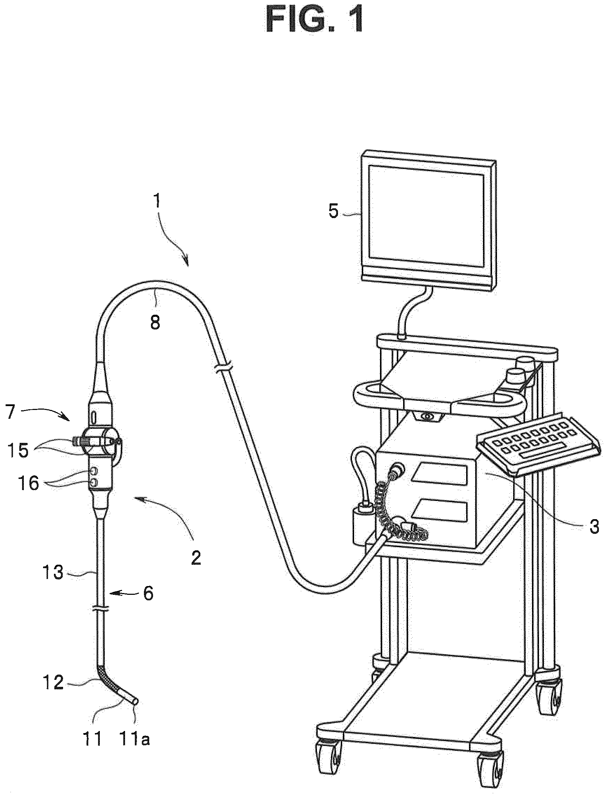

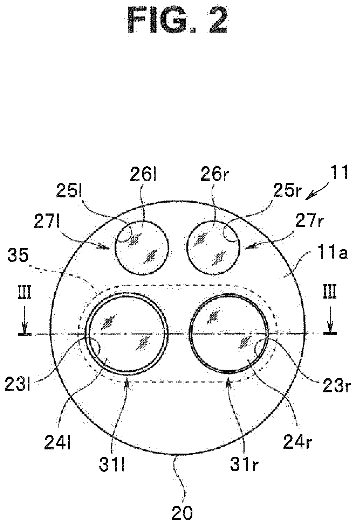

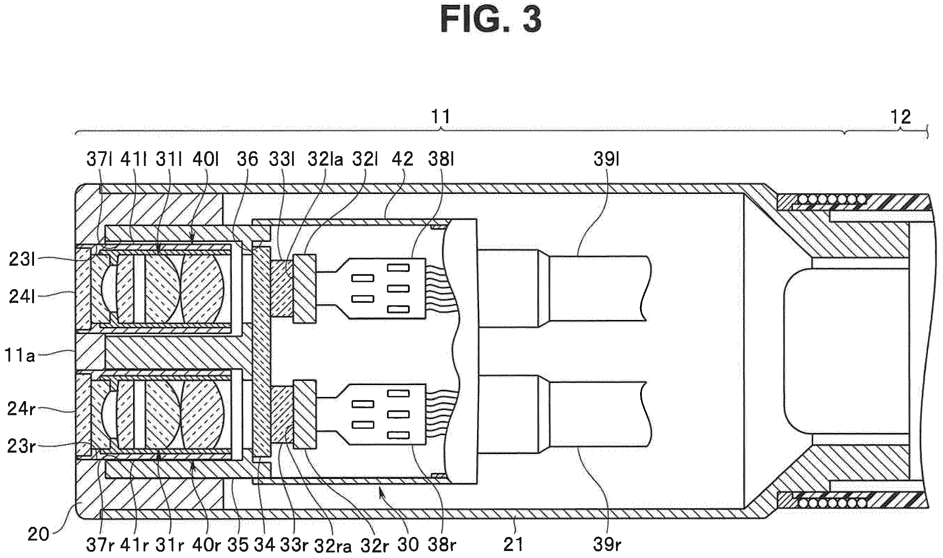

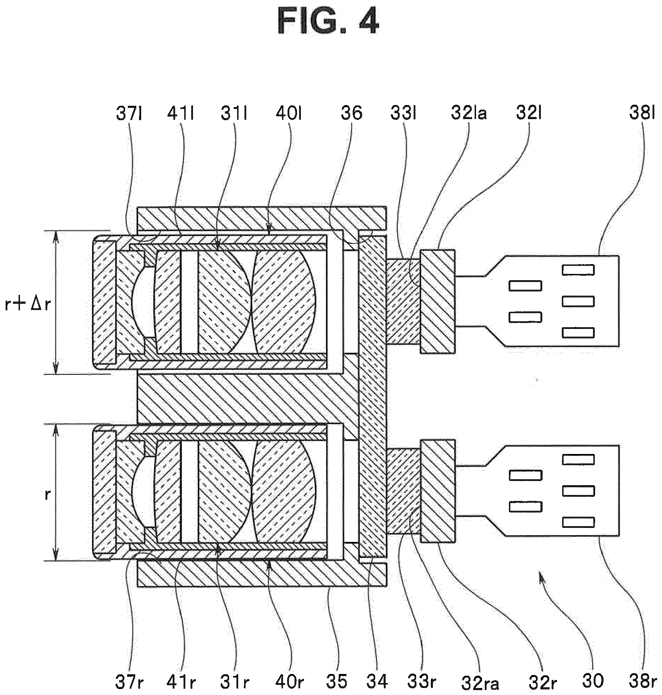

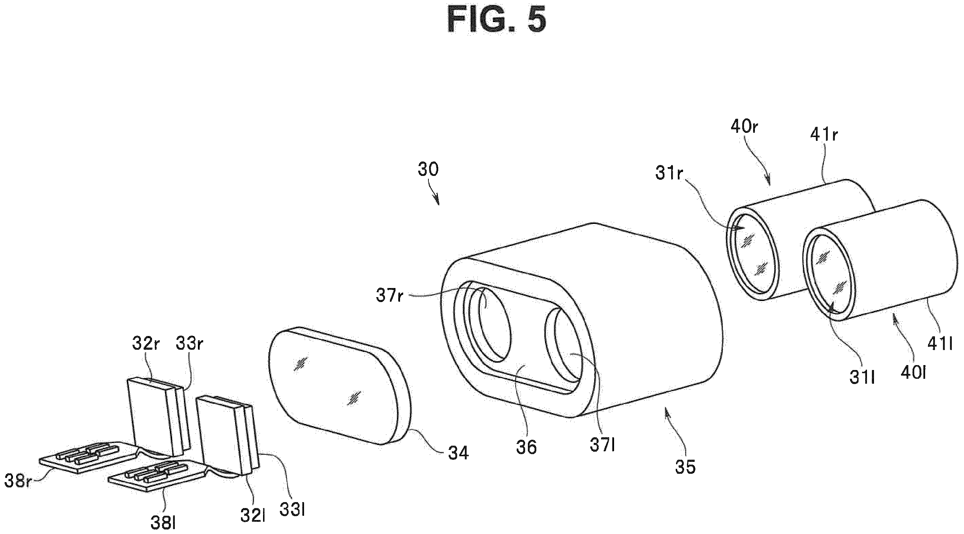

An embodiment of the present invention is described below with reference to drawings. The drawings relate to the embodiment of the present invention. Fig. 1 is a perspective view illustrating an entire configuration of an endoscope system. Fig. 2 is an end surface view of a distal end portion of an endoscope. Fig. 3 is a cross-sectional diagram taken along line III-III in Fig. 2. Fig. 4 is an enlarged cross-sectional diagram of a stereo image pickup unit. Fig. 5 is an exposed perspective view illustrating the stereo image pickup unit from proximal end side.

An endoscope system 1 illustrated in Fig. 1 includes a stereoscopic endoscope 2, a processor 3, and a monitor 5. The stereoscopic endoscope 2 stereoscopically picks up an image of a subject from different viewpoints. The stereoscopic endoscope 2 is detachably connected to the processor 3. The monitor 5 serves as a display apparatus that displays an image signal generated by the processor 3, as an endoscope image.

The stereoscopic endoscope 2 according to the present embodiment is, for example, a rigid endoscope applied to laparoscopic surgeries. The stereoscopic endoscope 2 includes an elongated insertion section 6, an operation section 7, and a universal cable 8. The operation section 7 is provided continuously to proximal end side of the insertion section 6. The universal cable 8 extends from the operation section 7 and is connected to the processor 3.

The insertion section 6 mainly includes a distal end portion 11, a bending portion 12, and a rigid tube portion 13 that are continuously provided in order from distal end side. The distal end portion 11 is configured of a metal member made of stainless steel or the like. The rigid tube portion 13 is configured of a metal tube made of stainless steel or the like.

The insertion section 6 is a section to be inserted into a body. A stereo image pickup unit 30 (see Fig. 3 and the like) that stereoscopically picks up an image inside the subject is incorporated in the distal end portion 11. Further, image pickup cable bundles 391 and 39r (see Fig. 3), a light guide bundle (not illustrated), and the like are inserted into the bending portion 12 and the rigid tube portion 13. The image pickup cable bundles 391 and 39r are electrically connected to the stereo image pickup unit 30. The light guide bundle transmits illumination light to the distal end portion 11. Note that, as the stereoscopic endoscope 2 according to the present embodiment, a rigid endoscope in which the proximal end side of the bending portion 12 is configured of the rigid tube portion 13 is exemplified; however, the stereoscopic endoscope 2 is not limited to the rigid endoscope, and the stereoscopic endoscope 2 may be a flexible endoscope in which the proximal end side of the bending portion 12 is configured of a flexible tube portion having flexibility.

An angle lever 15 to remotely operate the bending portion 12 is provided on the operation section 7, and various kinds of switches 16 to operate a light source apparatus of the processor 3, a video system center, and the like are further provided on the operation section 7.

The angle lever 15 is bending operation means that bendably operates the bending portion 12 of the insertion section 6 in four directions of up, down, right, and left, in this case. Note that the bending portion 12 is not limited to the configuration bendable in the four directions of up, down, right, and left, and may be configured to be bendably operated in, for example, two directions of only up and down, or only right and left.

Next, a configuration of the distal end portion of such a stereoscopic endoscope 2 is described in detail with reference to Figs. 2 and 3.

As illustrated in Fig. 3, the distal end portion 11 includes a distal end portion body 20 having a substantially columnar shape, and a distal end cylinder 21 that has a substantially cylindrical shape and has a distal end fixed to the distal end portion body 20. Here, the distal end of the distal end cylinder 21 is fitted to an outer periphery of the distal end portion body 20, and a distal end surface 11a of the distal end portion 11 is formed of an end surface of the distal end portion body 20 exposed from the distal end cylinder 21.

As illustrated in Figs. 2 and 3, paired observation through holes 231 and 23r that open on the distal end surface 11a are provided side by side (in other words, in a lateral bending direction of the bending portion 12) on the distal end portion body 20. Paired objective optical systems (first and second objective optical systems 311 and 31r) that configure the stereo image pickup unit 30 are respectively held by the left and right observation through holes 231 and 23r. This forms observation windows 241 and 24r on the distal end surface 11a of the distal end portion 11.

In addition, for example, as illustrated in Fig. 2, paired illumination through holes 251 and 25r that open on the distal end surface 11a are provided side by side on upper side of the observation through holes 231 and 23r (in other words, on upper side in a vertical bending direction of the bending portion 12) on the distal end portion body 20. Paired illumination optical systems 271 and 27r that are optically connected to the unillustrated light guide bundle are respectively held by the left and right illumination through holes 251 and 25r. This forms illumination windows 261 and 26r on the distal end surface 11a of the distal end portion 11.

As illustrated in Fig. 3 to Fig. 5, the stereo image pickup unit 30 includes: a first image pickup device 321 that receives an optical image (a first optical image) formed by the first objective optical system 311; a second image pickup device 32r that receives an optical image (a second optical image) formed by the second objective optical system 31r; a single centering glass 34 that is disposed on optical paths of the respective first and second optical images, and serves as an optical member to which light receiving surfaces 321a and 32ra of the respective first and second image pickup devices 321 and 32r are aligned and fixed through bonding; and a holding frame 35 that holds the first and second image pickup devices 321 and 32r through the centering glass 34.

Each of the first and second image pickup devices 321 and 32r is configured of a solid-state image pickup device such as a CCD (charge coupled device) and a CMOS (complementary metal oxide semiconductor). Cover glasses 331 and 33r to respectively protect the light receiving surfaces 321a and 32ra are respectively bonded to the first and second image pickup devices 321 and 32r.

Further, flexible print circuit boards (FPC boards) 381 and 38r are respectively electrically connected to terminal parts (not illustrated) provided on the first and second image pickup devices 321 and 32r. For example, various kinds of electronic components such as a digital IC to generate a driving signal of the image pickup device, an IC driving power-supply stabilization capacitor to stabilize a driving power supply of the digital IC, and a resistor are mounted on each of the FPC boards 381 and 38r through soldering, etc. In addition, the image pickup cable bundles 391 and 39r are respectively electrically connected to the FPC boards 381 and 38r.

Note that, in the present embodiment, the first and second image pickup devices 321 and 32r, the FPC boards 381 and 38r on which the various kinds of electronic components are mounted, and distal ends of the respective image pickup cables 391 and 39r that are respectively electrically connected to the FPC boards 381 and 38r are integrally covered with a single cover body 42.

The centering glass 34 is configured of a transparent glass substrate that extends in the lateral direction of the distal end portion 11. The light receiving surfaces 321a and 32ra of the first and second image pickup devices 321 and 32r are fixed to the centering glass 34 through the cover glasses 331 and 33r, respectively.

More specifically, the cover glasses 331 and 33r respectively adhered to the light receiving surfaces 321a and 32ra are bonded to the centering glass 34 with an ultraviolet curable transparent adhesive (a UV adhesive) or the like. As a result, the first and second image pickup devices 321 and 32r are positioned and fixed to the centering glass 34 while being separated from each other with a predetermined interval. Further, a distal end of the cover body 42 is fixed to a glass holding portion 36.

The holding frame 35 is configured of, for example, a columnar metal member that has a substantially round cornered rectangular cross-sectional surface (for example, see Fig. 5). The glass holding portion 36 is recessed on the proximal end side of the holding frame 35, and the centering glass 34 is fixed to the glass holding portion 36 with an adhesive or the like.

Further, for example, as illustrated in Figs. 3 and 4, a first objective optical system holding hole 371 and a second objective optical system holding hole 37r are provided side by side with a preset interval in between, on the holding frame 35. Distal ends of the respective first and second objective optical system holding holes 371 and 37r are opened on an end surface (the distal end surface 11a) of the holding frame 35, and proximal ends are configured of through holes that communicate with the glass holding portion 36.

The first and second objective optical systems 311 and 31r are respectively held by the first and second objective optical system holding holes 371 and 37r while being unitized as first and second objective optical system units 401 and 40r.

In other words, the first and second objective optical systems 311 and 31r are respectively held by first and second lens frames 411 and 41r, thereby respectively configuring the first and second objective optical system units 401 and 40r. Further, the first and second objective optical system units 401 and 40r are respectively positioned and fixed inside the first and second objective optical system holding holes 371 and 37r with an adhesive or the like, which causes the first and second objective optical systems 311 and 31r and the first and second image pickup devices 321 and 32r to be integrally held by the single holding frame 35.

In this case, to correct a slight machining error, a slight assembly error, and the like of each portion to optimize relative positions (such as a distance between optical axes and tilt angles) of the first and second objective optical systems 311 and 31r, one of the first and second objective optical system holding holes 371 and 37r (for example, the first objective optical system holding hole 371) is configured of a through hole larger in diameter than the other hole (for example, the second objective optical system holding hole 37r). In other words, for example, as illustrated in Fig. 4, in the present embodiment, the first objective optical system holding hole 371 is configured of a through hole having an inner diameter that is obtained by adding a predetermined adjustment margin Δr to an inner diameter r that receives the first lens frame 411 substantially without a gap. On the other hand, the second objective optical system holding hole 37r is configured of a through hole having the inner diameter r that receives the second lens frame 41r substantially without a gap. Further, the first objective optical system unit 401 is bonded and fixed inside the first objective optical system holding hole 371 while the distance between the optical axes, the tilt angle, and the like are slightly adjusted with respect to the second objective optical system unit 40r.

Next, an example of a method of assembling the stereo image pickup unit 30 having such a configuration is described.

In the method of assembling the stereo image pickup unit 30, first, the first and second image pickup devices 321 and 32r are positioned and fixed to the centering glass 34 through the UV adhesive.

In this case, the relative positions of the first and second image pickup devices 321 and 32r on the centering glass 34 are accurately positioned (centered) through, for example, observation of photoelectric conversion devices and the like disposed on the light receiving surfaces 321a and 32ra of the respective first and second image pickup devices 321 and 32r through the centering glass 34 under a microscope. Thereafter, in the positioned state in the above-described manner, ultraviolet rays are applied to cure the UV adhesive, which fixes the first and second image pickup devices 321 and 32r (more specifically, the cover glasses 331 and 33r) to the centering glass 34.

In a next step, the centering glass 34 holding the first and second image pickup devices 321 and 32r is fixed to the glass holding portion 36 provided on the holding frame 35, with an adhesive or the like. At this time, the centering glass 34 is adjusted such that, for example, the second image pickup device 32r is positioned at a predetermined position with respect to the second objective optical system holding hole 37r that does not include the adjustment margin Δr.

In a next step, the second objective optical system unit 40r is positioned and fixed inside the second objective optical system holding hole 37r. In other words, the second objective optical system unit 40r inserted into the second objective optical system holding hole 37r is adjusted in the optical axis direction (is focused) under observation of the second optical image formed on the second image pickup device 32r, and is then fixed with an adhesive or the like.

In next step, the first objective optical system unit 401 is positioned and fixed inside the first objective optical system holding hole 371. In other words, the first objective optical system unit 401 inserted into the first objective optical system holding hole 371 is adjusted in the optical axis direction (is focused) under observation of the first optical image formed on the first image pickup device 321. Further, the first objective optical system unit 401 is adjusted in relative position (such as the distance between the optical axes and the tilt angle) with respect to the second objective optical system unit 40r within a range of the adjustment margin set in the first objective optical system holding hole 371, based on comparison between the first optical image formed on the first image pickup device 321 and the second optical image formed on the second image pickup device 32r. The first objective optical system unit 401 is then fixed with the adhesive or the like.

According to such an embodiment, the respective light receiving surfaces 321a and 32ra of the first image pickup device 321 that receives the first optical image formed by the first objective optical system 311 and the second image pickup device 32r that receives the second optical image formed by the second objective optical system 31r, are positioned and fixed, with an adhesive or the like, to the single centering glass 34 that is disposed on the optical paths of the respective first and second optical images. The first and second image pickup devices 321 and 32r are held by the holding frame 35 through the centering glass 34. As a result, it is possible to acquire two picked-up images with appropriate parallax through a simple configuration.

In other words, the first and second image pickup devices 321 and 32r are positioned and fixed to the single centering glass 34 and are held by the holding frame 35, which makes it possible to reduce the number of components to simplify the configuration, as compared with a case where the first and second image pickup devices are positioned and fixed to the holding frame through individual centering glasses. Further, the relative positions of the first and second image pickup devices 321 and 32r are determined on the single centering glass 34 that is smaller in thermal expansion and thermal contraction than the metal holding frame 35, which makes it possible to accurately position the first and second image pickup devices 321 and 32r at appropriate parallax positions. Therefore, it is possible to acquire two picked-up images with appropriate parallax. In addition, in the configuration in which the first and second image pickup devices 321 and 32r are fixed to the single centering glass 34, it is possible to prevent the distance between the optical axes of the first and second image pickup devices 321 and 32r from becoming excessively large, and to prevent three-dimensional appearance in stereoscopic observation from becoming excessively high, as compared with a configuration in which the first and second image pickup devices 321 and 32r are fixed to individual centering glasses.

In this case, the first objective optical system holding hole 371 that holds the first objective optical system 311 and the second objective optical system holding hole 37r that holds the second objective optical system 31r are provided on the holding frame 35, which allows the single holding frame 35 to hold not only the first and second image pickup devices 321 and 32r but also the first and second objective optical systems 311 and 31r. This makes it possible to achieve further simplification of the configuration. In addition, providing the first and second objective optical system holding holes 371 and 37r on the single holding frame 35 makes it possible to prevent the distance between the optical axes from becoming excessively large.

Moreover, the first and second objective optical systems 311 and 31r are assembled to the holding frame 35 while being respectively held by the first and second lens frames 411 and 41r with high accuracy and respectively unitized as the first and second objective optical system units 401 and 40r. This makes it possible to improve attachment accuracy of the first and second objective optical systems 311 and 31r with respect to the holding frame 35.

Further, providing the adjustment margin in one of the first and second objective optical system holding holes 371 and 37r makes it possible to easily achieves relative positional adjustment of the first and second objective optical systems 311 and 31r, even when the first and second objective optical system holding holes 371 and 37r are provided on the single holding frame 35.

Note that the present invention may be variously modified and alternated without limitation to the above-described embodiment, and such modifications and alternations are also included in the technical scope of the present invention.

The present application is based upon and claims the benefit of priority of Japanese Patent Application No. 2015-246015 filed in Japan on December 17, 2015, the disclosed contents of which are incorporated in the specification and claims of the present application by reference.

## Claims

1. A stereo image pickup unit, comprising:
a first image pickup device configured to receive a first optical image formed by a first objective optical system;
a second image pickup device configured to receive a second optical image formed by a second objective optical system that is paired with the first objective optical system;
a single optical member that is disposed on optical paths of the respective first and second optical images and to which light receiving surfaces of the respective first and second image pickup devices are positioned and fixed through bonding; and
a holding frame that holds the first and second image pickup devices through the optical member.

2. The stereo image pickup unit according to claim 1, wherein the holding frame includes a first objective optical system holding hole that holds the first objective optical system and a second objective optical system holding hole that holds the second objective optical system.

3. The stereo image pickup unit according to claim 2, wherein
the first objective optical system holding hole holds the first objective optical system unitized as a first objective optical system unit, and
the second objective optical system holding hole holds the second objective optical system unitized as a second objective optical system unit.

4. The stereo image pickup unit according to claim 3, wherein one of the first objective optical system holding hole and the second objective optical system holding hole includes an adjustment margin that allows adjustment of a position of one of the first objective optical system unit and the second objective optical system unit with respect to another objective optical system unit.
